(19) Europäisches Patentamt

European Patent Office

Office européen des brevets

(11) Veröffentlichungsnummer: **0 284 946 B1**

(12) **EUROPÄISCHE PATENTSCHRIFT**

(45) Veröffentlichungstag der Patentschrift: **22.04.92**

(51) Int. Cl.⁵: **A61B 7/02**

(21) Anmeldenummer: **88104517.3**

(22) Anmeldetag: **22.03.88**

(54) **Stethoskop.**

(30) Priorität: **28.03.87 DE 8704663 U**

(43) Veröffentlichungstag der Anmeldung:
**05.10.88 Patentblatt 88/40**

(45) Bekanntmachung des Hinweises auf die
Patenterteilung:
**22.04.92 Patentblatt 92/17**

(84) Benannte Vertragsstaaten:
**DE ES FR GB IT**

(56) Entgegenhaltungen:
**WO-A-81/00353**
**AU-B- 504 509**
**DE-A- 2 607 990**
**DE-U- 6 928 831**
**DE-U- 8 704 663**

(73) Patentinhaber: **Kirchner & Wilhelm**
**Heusteigstrasse 70 A**
**W-7000 Stuttgart-1(DE)**

(72) Erfinder: **Hansjörg, Kirchner**
**Am Mühlberg 45**
**W-7145 Markgröningen(DE)**

(74) Vertreter: **Schmid, Berthold, Dipl.-Ing. et al**
**Kohler Schmid + Partner Patentanwälte**
**Ruppmannstrasse 27**
**W-7000 Stuttgart 80(DE)**

## Beschreibung

Die Erfindung betrifft ein Stethoskop mit einem Stethoskopbruststück, welches einen Schlauchanschlußkörper und wenigstens einen Schalltrichter oder ein Membranteil aufweist und der mit einem Schlauchanschlußstutzen verbindbar ist.

Die Fertigung einen solchen Stethoskops, genauer gesagt, des Bruststücks dieses Stethoskops, ist verhältnismäßig aufwendig und damit teuer. Aus hygienischen Gründen sind diese Stethoskopbruststücke aus Metall gefertigt (AU-B1-30003/77), weshalb sie relativ teuer sind. Es kommt noch hinzu, daß aufgrund des hohen Artgewichts der Metalle das Bruststück vergleichsweise schwer wird.

Mit der DE-U 69 28 831 ist ein Stehtoskopbruststück nach dem Oberbegriff des Anspruchs 1 bekannt, welches aus Kuststoff besteht. Zwar ist die Herstellung derartiger Bruststücke preiswerter, jedoch ergeben sich aufgrund des Kunststoffs hinsichtlich der Hygiene bzw. Sterilisation Probleme. Die schalltechnischen Eigenschaften werden ebenfalls nachhaltig beeinflußt.

Die Aufgabe der Erfindung besteht infolgedessen darin, ein Stethospkop der eingangs genannten Art so weiterzuentwickeln, daß sich sein Gewicht reduzieren und möglichst auch die Herstellungskosten senken lassen, jedoch die hygienischen und schalltechnischen Eigenschaften nicht beeinflußt werden.

Zur Lösung dieser Aufgabe wird erfindungsgemäß vorgeschlagen, daß das Stethoskopbruststück mehrteilig ausgebildet ist, wobei ein Teil von dem aus Kunststoff bestehenden Schlauchanschlußkörper und der andere Teil von dem aus Metall bestehenden Schalltrichter und/oder dem Membranteil gebildet werden, und daß die Teile mittels eines Befestigungselements miteinander verbunden sind.

Aus schalltechnischen Gründen gibt man weiterhin dem metallenen Schalltrichter oder Membranteil (ohne Membrane) den Vorzug vor einer Kunststoffausführung, jedoch ist Kunststoff für den Schlauchanschlußkörper gut geeignet und bekanntermaßen wesentlich leichter als Metall. Durch entsprechende Ausbildung der Spritzform zur Herstellung des Schlauchanschlußkörpers kann man dieses formlich so ausbilden, daß keine oder keine nennenswerte Bearbeitung erforderlich ist. Aufgrund der separaten Fertigung des Schalltrichters bzw. des Membranteils sind diese beiden Bestandteile des Stethoskops einfach zu gestalten, so daß man auch diesbezüglich Kosten, insbesondere für zerspanende Arbeit, einsparen kann. Bei diesem Stethoskop kann es sich sowohl um eine Ausführung handeln, die nur einen Schalltrichter aufweist, als auch eine andere Variante mit lediglich einem Membranteil. Schließlich ist die erfindungsgemäße Ausbildung auch für ein Stethoskop geeignet, das sowohl einen Schalltrichter als auch einen Membranteil trägt. Ein solches Stethoskop muß "umschaltbar" sein, um den Ohrbügel und den Verbindungsschlauch wahlweise mit dem Schalltrichter oder dem Membranteil akustisch bzw. schwingungsmäßig verbinden zu können. Insbesondere dieses Stethoskop kann durch die Verwendung eines kunststoffgefertigten Schlauchanschlußkörpers leichter und preiswerter gefertigt werden, als ein reines Metall-Stethoskop, das nicht unerhebliche Zerspanungsarbeit erfordert und auch schwerer ist.

Eine besonders bevorzugte Ausführungsform eines solchen Stethoskops mit einem Schalltrichter und einem Membranteil sowie einem drehbar und in zwei definierten Drehlagen verrastbaren, den Schlauchanschlußstutzen bildenden oder aufnehmenden Anschlußröhrchen ist dadurch gekennzeichnet, daß der Schalltrichter, das Membranteil bzw. dessen Grundkörper und das Anschlußröhrchen aus Metall gefertigt sind. Diese Metallteile werden in geeigneter Weise mit dem aus Kunststoff hergestellten, insbesondere gespritzten Schlauchanschlußkörper mittels der Befestigungselemente verbunden. Durch Drehen des Röhrchens um seine Längsachse oder, bei gekrümmter Ausführung, um die Längsachse des in den Schlauchanschlußkörper eingesteckten Endes erreicht man in der einen festgelegten Drehstellung die akkustische Verbindung mit dem Schalltrichter und in der anderen definierten Drehstellung die akkustische Verbindung mit dem Membranteil. Aus diesem Grunde ist das innere Ende dieses Anschlußröhrchens in bekannter Weise mit einer einzigen Radialbohrung versehen, die in der einen definierten Drehstellung mit einem weiterführenden Kanal des Schlauchanschlußkörpers zum Schalltrichter und in der anderen definierten Drehstellung mit einem anderen weiterführrenen Kanal des Schlauchanschlußkörpers zum Membranteil verbunden ist.

Das Anschlußröhrchen ist in Weiterbildung der Erfindung mittels eines Sperrglieds gegen Herausziehen aus seiner Lagerbohrung des Schlauchanschlußkörpers gesichert, wobei sich das abnehmbare Sperrglied an einer nach innen weisenden Anschlagfläche, einer Bohrung od. dgl. des Schlauchanschlußkörpers abstützt. Demnach wird also das Anschlußröhrchen zunächst in die hierfür vorgesehene Lagerbohrung des Schlauchanschlußkörpers hineingeschoben und anschließend montiert man dann das Sperrglied. Es fixiert das Anschlußröhrchen in axialer Richtung, hindert aber, zumindest im Falle eines "umschaltbaren" Stethoskops, dessen Drehung nicht.

Eine besonders bevorzugte Variante dieses Stethoskops kennzeichnet sich dadurch, daß das Sperrglied zugleich ein Verrastglied für die beiden Arbeits-Drehstellungen des Anschlußröhrchens bildet. Dies reduziert die Teilezahl und senkt somit die Herstellungkosten.

Weitere vorteilhafte Ausgestaltungen dieses Stethoskops und damit verbundene Vorteile ergeben sich aus der nachfolgenden Beschreibung eines Ausführungsbeispiels.

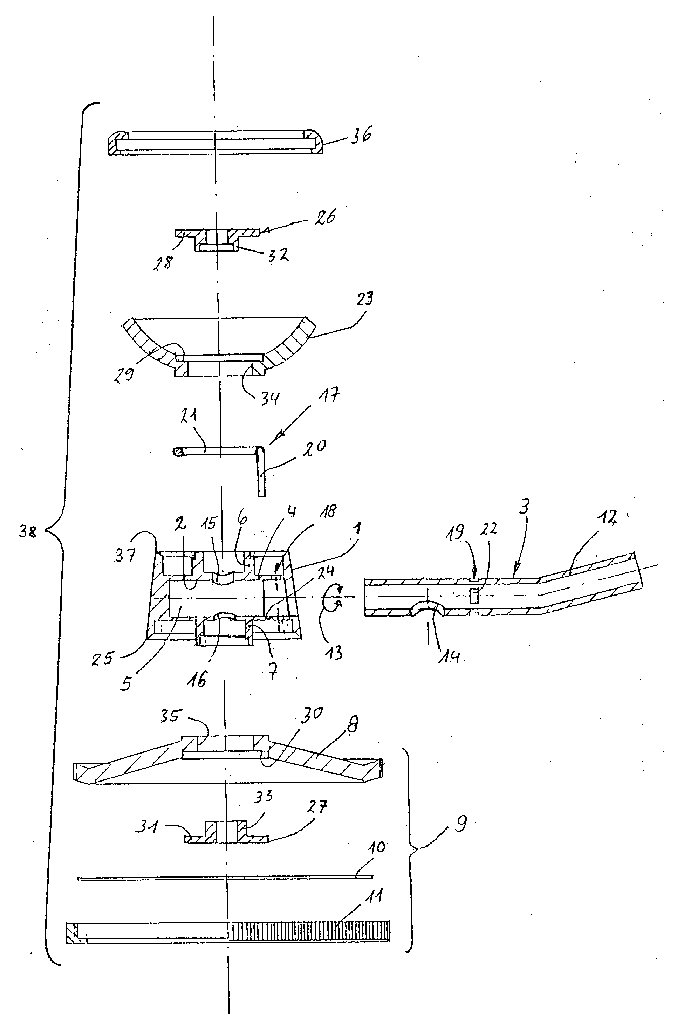

Die Zeichnung zeigt ein solches Ausführungsbeispiel. Es handelt sich um eine explosionsartige Darstellung der einzelnen Teile des Bruststücks vor dem Zusammenbau, wobei die Teile im Längsmittelschnitt, teilweise auch im Halbschnitt gezeichnet sind.

Die in der Zeichnung dargestellten einzelnen Teile bilden, wie gesagt, zusammen das Bruststück 38 des Stethoskops. Der Großteil davon ist nach der Montage zu einer bleibenden Einheit zusammengefasst, wie nachstehend noch erläutert wird. Ein wesentliches Teil dieses Stethoskops, bzw. des Bruststücks 38, ist der Schlauchanschlußkörper 1. Es handelt sich dabei um ein aus Kunststoff hergestelltes, vorzugsweise in einer Form gespritztes Teil. Es besitzt eine radiale Bohrung 2, in welches das eine Ende eines vorzugsweise leicht abgewinkelten Anschlußröhrchens 3 mit Spiel eingesteckt wird, dessen, in der Zeichnung rechtes Ende, einen Schlauchanschlußstutzen 12 bildet. Der Schlauch schafft die Verbindung zum nicht dargestellten Ohrbügel. Das Schlauchanschlußstück 1 besteht im wesentlichen aus einem konischen Ring mit einer Zwischenwand 4, einem an deren Unterseite angeformten, tunnelförmigen Lageransatz 5 sowie zwei nach entgegengesetzten Seiten abstehenden Zentrieransätzen 6 und 7. Der obere Zentrieransatz 6 ist an die Zwischenwand 4 angeformt während sich der untere Zentrieransatz 7 vom Lageransatz 5 aus nach unten erstreckt.

Auf den Zentrieransatz 6 wird ein Schalltrichter 23 aufgesteckt, während der Zentrieransatz 7 den ebenfalls etwa trichterförmigen Grundkörper 8 des Membrateils 9 aufnimmt. Das Membranteil 9 umfaßt den Grundkörper 8, die Membrane 10 und deren Befestigungsring 11, welcher auf das Außengewinde des Grundkörpers 8 aufgeschraubt wird. Der Befestigungsring 11 und der Grundkörper 8 sind aus Metall, beispielsweise aus Messing, Bronze od. dgl.. Das Anschlußröhrchen 3 ist vorzugsweise auch aus Messing gefertigt und dasselbe gilt für den Trichter 8. Diese Teile können verchromt sein.

Wenn das Bruststück 38 nicht lediglich nur einen Schalltrichter 23 oder nur ein Mebranteil 9 aufweist, sondern mit beidem ausgestattet ist, so muß es "umschaltbar" ausgebildet sein. Zu diesem Zwecke ist das Anschlußröhrchen 3, wie bereits erläutert, mit Spiel in die Bohrung 2 eingeschoben und kann darin im Sinne des Doppelpfeils 13, vorzugsweise um 360°, gedreht werden. Es besitzt allerdings, wie nachstehend noch näher erläutert wird, zwei definierte Drehstellungen, in denen entweder der Schalltrichter 23 oder das Membranteil 9 akustisch "angeschlossen" sind. Das Anschlußröhrchen 3 besitzt eine radiale Bohrung 14, die in der einen definierten Drehstellung einer Querbohrung 15 der Zwischenwand 14 zugeordnet ist und in der anderen, um 180° versetzt definierten Drehstellung einer Querbohrung 16 des tunnelförmigen Lageransatzes 5. In der gezeichneten Lage des Anschlußröhrchens 3 ist der Membranteil 9 "angeschlossen".

Damit das Anschlußröhrchen 3 in der zusammengebauten Stellung des Bruststücks 38 aus dem Schlauchanschlußkörper nicht herausgezogen werden kann, wird es mit Hilfe eines Sperrglieds 17 in axialer Richtung gehalten. Dieses ist zugleich auch als Verrastglied für die beiden Arbeits-Drehstellungen des Anschlußröhrchens 3 ausgebildet. Dabei arbeitet es mit einer Außen-Ringnut 19 des Anschlußröhrchens 3 zusammen. Diese ist einer Bohrung 18 oder einem Durchbruch der Zwischenwand 4 zugeordnet. Beim Ausführungsbeispiel ist ein Bohrungspaar vorgesehen. Durch letzteres ragen die beiden parallelen Schenkel 20 des Sperr- und Verrastglieds 17 hindurch. Sie liegen tangential an zwei um 180° versetzten Stellen am Grund der Ringnut 19 an. Genauer gesagt werden sie an diesen Grund der Ringnut federelastisch angepreßt, weil das Sperr- und Verrastglied nach Art einer Bügelfeder ausgebildet ist. Der ringförmige Teil 21 preßt die abgewinkelten Enden, welche die beiden Schenkel 20 bilden, federelastisch gegeneinander bzw. nach der Montage gegen den Grund der Ringnut 19. Damit läßt sich das Anschlußröhrchen 3 in der Bohrung 2 drehen, aber in axialer Richtung nicht aus der Bohrung 2 herausziehen.

Um nun auch mit Hilfe dieses Sperr- und Verrastglieds 17 die beiden definierten Arbeits-Drehstellungen des Anschlußröhrchens 3 zu bekommen, befindet sich am Grund der Ringnut 19 eine Abflachung 22, welche beim Ausführungsbeispiel gegenüber der Radialbohrung 14 des Anschlußröhrchens 3 um 90° versetzt ist. Wegen der Dünnwandigkeit des Röhrchens 3 ist beim Ausführungsbeispiel an Stelle der Abflachung 22 ein fensterartiger Durchbruch entstanden. Er ist aber in seiner Wirkung mit einer Abflachung identisch. Der zugeordnete Schenkel, beispielsweise der in der Zeichnung des geschnittenen Sperrglieds 17 zu sehende Schenkel 20, legt sich in der dargestellten Drehlage des Anschlußröhrchens 3 aufgrund seiner Federkraft an diese Abflachung bzw. dieses Fenster an, so daß eine leichte Drehsperre entsteht. Dreht man das Röhrchen nunmehr um 180° -wobei die Drehrichtung keine Rolle spielt- so wird die Abflachung 22 bzw. der Durchbruch dem anderen Schenkel 20 zugeordnet, was man durch ein leichtes Einrasten feststellen kann.

Die Radialbohrung 14 ist jetzt der Querbohrung 15 zugeordnet und damit kann der Schalltrichter 7 des Stethokops für die Untersuchung herangezogen werden.

Beim Ausführungsbeispiel befindet sich das Sperrglied 17 zwischen dem Schlauchanschlußkörper 1 oder genauer gesagt zwischen dessen Zwischenwand 4 und dem Schalltrichter 23. Es ist ohne weiteres einzusehen, daß man das Sperr- und Verrastglied 17 auch zwischen den Schlauchanschlußkörper 1 und den Grundkörper 8 des Membranteils 9 einsetzen kann. Im übrigen befindet sich unterhalb der Bohrung 18 od. dgl. eine weitere Bohrung oder ein weiteres Bohrungspaar 23 an einem Verbindungssteg 24. Stattdessen können sich die beiden freien Schenkelenden sich auch beidseits eines schmalen Verbindungsstegs 24 erstrecken. Letzterem kommt aber eher aus spritztechnischen Gründen Bedeutung zu.

In zusammengebautem Zustand liegt die Unterseite des Schalltrichters 23 auf dem nach oben weisenden Stützrand 37 des Schlauchanschlußkörpers 1 auf. Am gegenüberliegenden Stützrand 25 liegt der Grundkörper des Membranteil 9 an. Diese Teile werden mit Hilfe von hohlnietartigen Befestigungselementen 26 bzw. 27 drehfest niedergehalten. Der Bund 28 des Befestigungselements 26 wird von einer Ausdrehung 29 des Schalltrichters 23 aufgenommen, eine entsprechende Ausdrehung 30 des Grundkörpers 8 nimmt den Bund 31 des Befestigungselements 27 auf. Der Schaft 32 wird durch Ultraschallschweißung mit dem Zentrieransatz 6 des Schlauchanschlußkörpers 1 bleibend verbunden. Entsprechend findet eine bleibende Verbindung des Schaftes 33 des Befestigungselements 27 mit dem Zentrieransatz 7 statt. Um ein Drehen des Schalltrichters 23 bzw. des Grundkörpers 8 zu verhindern, kann deren Bohrung 34 bzw. 35 unrund sein, insbesondere eine Sechskantform aufweisen. Dementsprechend ist dann auch die Außenkontur der Zentrieransätze 6 und 7 zu wählen. Im übrigen kann man noch weitere Zentrierungen vorsehen, wie dies beim Zentrieransatz 6 und Befestigungselement 26 angedeutet ist. Auf den Außenrand des Schalltrichters 3 wird in bekannter Weise ein Kunststoffring 36 aufgesprengt, der das Aufsetzen des Schalltrichters 23 auf den Körper angenehmer gestaltet.

## Patentansprüche

1. Stethoskop mit einem Stethoskopbruststück (38), welches einen Schlauchanschlußkörper (1) und wenigstens einen Schalltrichter (23) und oder ein Membranteil (9) aufweist und mit einem Schlauchanschlußstutzen (12) verbindbar ist, dadurch gekennzeichnet, daß das Stethoskopbruststück (38) mehrteilig ausgebidet ist, wobei ein Teil von dem aus Kunststoff bestehenden Schlauchanschlußkörper (1) und der andere Teil von dem aus Metall bestehenden Schalltrichter (23) und/oder dem aus Metall bestehenden Grundkörper des Membranteils (9) gebildet werden und daß die Teile mittels eines Befestigungselements (26, 27) miteinander verbunden sind.

2. Stethoskop nach Anspruch 1 mit einem Schalltrichter (23) und einem Membranteil (9) sowie einem drehbar und in zwei definierten Drehlagen verrastbaren, den Schlauchanschluß bildenden oder aufweisenden metallenen Anschlußröhrchen (3), welches mittels eines Sperrglieds (17) in einer Lagerbohrung (2) des Anschlußkörpers (1) axial gehalten und in zwei um 180° versetzten Drehlagen verrastbar ist, dadurch gekennzeichnet, daß das Sperrglied (17) zugleich ein Verrastglied für die beiden Arbeits-Drehstellungen des Anschlußröhrchens (3) bildet.

3. Stethoskop nach Anspruch 2, dadurch gekennzeichnet, daß das Anschlußröhrchen (3) mit einer Ringnut (19) versehen ist, in welche zwei federelastisch gegeneinander gedrückte, etwa parallele Schenkel (20) des Sperr- und Verrastglieds (17) eingreifen, wobei sich das Anschlußröhrchen (3) zwischen den Schenkeln (20) befindet und seine Ringnut (19) an ihrem Grund eine Abflachung (22) aufweist.

4. Stethoskop nach Anspruch 3, dadurch gekennzeichnet, daß das Sperr- und Verrastglied (17) in der Art einer Bügelfeder ausgebildet ist und sie sich zwischen des Schlauchanschlußkörper (1) und dem Schalltrichter (23) oder dem Membranteil (9) befindet, wobei ihre abgewinkelten freien Schenkelenden (20) in die Ringnut (19) des Anschlußröhrchens (3) tangential eingreifen.

5. Stethoskop nach Anspruch 4, dadurch gekennzeichnet, daß der Schlauchanschlußkörper (1) eine sich etwa parallel zur Membrane (10) erstreckende Zwischenwand (4) aufweist, auf welcher der ringförmige Hauptteil (21) des Sperr- und Verrastglieds (17) aufliegt, wobei die abgewinkelten freien Schenkelenden (20) einen Durchbruch, Bohrungen (18) der Zwischenwand durchsetzen.

6. Stethoskop nach Anspruch 5, gekennzeichnet durch einen tunnelförmigen, an die Zwischenwand (4) angeformten Lageransatz (5) für das eingesteckte Ende des Anschlußröhrchens (3).

7. Stethoskop nach Anspruch 6, dadurch gekennzeichnet, daß sich beidseits der Zwischenwand (4) ein Zentrieransatz (6, 7) für den Schalltrichter (23) einerseits und den Membranteil (9) andererseits befindet.

8. Stethoskop nach Anspruch 7, dadurch gekennzeichnet, daß der Schalltrichter (23) und ein Grundkörper (8) des Membranteils (9) zwischen jeweils einem Stützrand (24, 25) des Schlauchanschlußkörpers (1) und einem Bund (28, 31) eines hohlnietartigen Befestigungselements (26, 27) eingespannt ist, wobei das Befestigungselement mit dem zugeordneten Zentrieransatz (6, 7) fest verbunden ist.

9. Stethoskop nach Anspruch 8, dadurch gekennzeichnet, daß der Zentrieransatz (6, 7) und das Befestigungselement (26, 27) jeweils steckbar miteinander verbunden und durch Ultraschallschweißung zusammengehalten sind.

## Claims

1. Stethoscope which has a stethoscope breast piece (38) which comprises a tube connection body (1) and at least one sound funnel (23) and/or a membrane section (9) and can be connected to a tube connection piece (12), characterised in that the stethoscope breast piece (38) is formed in a number of parts, wherein one part is formed by a synthetic material tube connection body (1) and the other part is formed from a metal sound funnel (23) and/or the metal base body of the membrane section (9); and in that the parts are connected to one another by means of a fastening element (26, 27).

2. Stethoscope according to Claim 1 having a sound funnel (23) and a membrane section (9) as well as a rotatable metal small connection tube (3) which can be locked in two defined rotational positions, forms or comprises the tube connection, is axially retained by means of a blocking member (17) in a bearing bore (2) of the connection body (1) and can be locked in two rotational positions offset by 180°, characterised in that the blocking member (17) also forms a locking member for the two rotational operating positions of the small connection tube (3).

3. Stethoscope according to Claim 2, characterised in that the small connection tube (3) is provided with an annular groove (19) in which there engage two legs (20) of the blocking and locking member (17), which are pressed against one another in a resilient manner and are approximately parallel, wherein the small connection tube (3) is located between the legs (20) and its annular groove (19) comprises a flattened portion (22) on its base.

4. Stethoscope according to Claim 3, characterised in that the blocking and locking member (17) is formed in the manner of a clamping spring and is located between the tube connection body (1) and the sound funnel (23) or the membrane section (9), wherein its angled free leg ends (20) engage tangentially in the annular groove (19) of the small connection tube (3).

5. Stethoscope according to Claim 4, characterised in that the tube connection body (1) comprises an intermediate wall (4) which extends approximately parallel to the membrane (10) and on which the annular main section (21) of the blocking and locking member (17) rests, wherein the angled free leg ends (20) penetrate an aperture, bores (18) in the intermediate wall (4).

6. Stethoscope according to Claim 5, characterised by a tunnel-shaped bearing surface (5) which is formed on the intermediate wall (4) for the inserted end of the small connection tube (3).

7. Stethoscope according to Claim 6, characterised in that a centring attachment (6, 7) is located on both sides of the intermediate wall (4) for the sound funnel (23) on the one hand and the membrane section (9) on the other.

8. Stethoscope according to Claim 7, characterised in that the sound funnel (23) and a base body (8) of the membrane section (9) are clamped in between a supporting edge (24, 25) of the tube connection body (1) and a collar (28, 31) of a fastening element (26, 27) in the form of a hollow-type rivet in each case, wherein the fastening element is rigidly connected to the associated centring attachment (6, 7).

9. Stethoscope according to Claim 8, characterised in that the centring attachment (6, 7) and the fastening element (26, 27) are in each case connected to one another in a pluggable manner and are held together by means of ultrasonic welding.

## Revendications

**1.** Stéthoscope, ayant une pièce de poitrine (38) comprenant un corps (1) d'embranchement de tuyau flexible et au moins un cornet (23) et/ou un élément (9) à diaphragme et pouvant être raccordé à une tubulure (12) de branchement de tuyau flexible, caractérisé en ce que la pièce de poitrine (38) du stéthoscope est composée de plusieurs pièces, dont une partie est constituée par le corps (1) d'embranchement de tuyau flexible, réalisé en matière plastique, et dont l'autre partie est constituée par le cornet métallique (23) et/ou par le corps métallique de base de l'élément (9) à diaphragme, et en ce que les pièces sont reliées les unes aux autres par l'intermédiaire d'un élément (26, 27) de fixation.

**2.** Stéthoscope selon la revendication 1, comprenant un cornet (23) et un élément (9) à diaphragme, ainsi qu'un petit tube métallique (3) de raccordement, pouvant tourner et pouvant être encliqueté dans deux positions définies de rotation, constituant ou recevant l'élément de raccordement pour le tuyau flexible, ce petit tube étant maintenu axialement dans un alésage (2) de logement du corps (1) d'embranchement, au moyen d'un élément (17) de verrouillage, et pouvant s'encliqueter dans deux positions de rotation décalées de 180°, caractérisé en ce que l'élément (17) de verrouillage constitue en même temps un élément d'encliquetage pour les deux positions de travail, obtenues par rotation, du petit tube (3) de raccordement.

**3.** Stéthoscope selon la revendication 2, caractérisé en ce que le petit tube (3) de raccordement est pourvu d'une gorge circulaire (19), dans laquelle s'engagent deux branches à peu près parallèles (20) de l'élément (17) de verrouillage et d'encliquetage, le petit tube (3) de raccordement se trouvant situé entre les deux branches (20) et la gorge circulaire (19) de celui-ci comportant dans son fond un méplat (22).

**4.** Stéthoscope selon la revendication 3, caractérisé en ce que l'élément (17) de verrouillage et d'encliquetage est réalisé sous forme d'un étrier élastique, et en ce qu'il se situe entre le corps (1) d'embranchement de tuyau flexible et le cornet (23) ou l'élément (9) à diaphragme, les branches repliées libres (20) de l'élément de fixation s'engageant tangentiellement dans la gorge circulaire (19) du petit tube (3) de raccordement.

**5.** Stéthoscope selon la revendication 4, caractérisé en ce que le corps (1) d'embranchement de tuyau flexible comporte une paroi intermédiaire (4) disposée à peu près parallèlement au diaphragme (10), sur laquelle repose la partie annulaire principale (21) de l'élément (17) de verrouillage et d'encliquetage, les extrémités libres des branches repliées (20) traversant une lumière ou des trous (18) de la paroi intermédiaire.

**6.** Stéthoscope selon la revendication 5, caractérisé par un bossage (5) de logement, en forme de tunnel, façonné sur la paroi intermédiaire (4), destiné à recevoir l'extrémité insérée du petit tube (3) de raccordement.

**7.** Stéthoscope selon la revendication 6, caractérisé en ce qu'un bossage (6, 7) de centrage se situe de part et d'autre de la paroi intermédiaire (4), pour recevoir d'une part le cornet (23) et d'autre part l'élément (9) à diaphragme.

**8.** Stéthoscope selon la revendication 7, caractérisé en ce que le cornet (23) et un corps (8) de base de l'élément (9) à diaphragme sont serrés respectivement entre un bord (24, 25) d'appui du corps (1) d'embranchement de tuyau flexible et une collerette (28, 31) d'un élément (26, 27) de fixation en forme de rivet creux, l'élément de fixation étant relié fermement au bossage de centrage correspondant (6, 7).

**9.** Stéthoscope selon la revendication 8, caractérisé en ce que le bossage (6, 7) de centrage et l'élément (26, 27) de fixation sont reliés l'un à l'autre par emboîtement et sont fixés l'un sur l'autre par soudage par ultrasons.